# EUROPEAN PATENT SPECIFICATION

(11) **EP 4 322 867 B1**
(45) Date of publication and mention of the grant of the patent: **22.04.2026**
(21) Application number: 22717248.3
(22) Date of filing: 12.04.2022
(51) Int. Cl.: A61B 17/12, A61B 17/132

(54) **A SYSTEM FOR TREATING PENETRATING WOUNDS**
SYSTEM ZUR BEHANDLUNG EINDRINGENDER WUNDEN
SYSTÈME POUR LE TRAITEMENT DE PLAIES PÉNÉTRANTES

(30) Priority: 13.04.2021 GB 202105238
(43) Date of publication of application: 21.02.2024
(73) Proprietor: Act Medical Ltd, LE11 3UZ (GB)
(72) Inventor: BENTLEY, Joseph, Witham CM8 1DZ (GB)
(74) Representative: Appleyard Lees IP LLP
(86) International application number: PCT/GB2022/050907
(87) International publication number: WO 2022/219316

(56) References cited:
- EP-A1- 2 896 413
- WO-A1-2017/181747
- CN-A- 108 969 047
- US-A1- 2005 143 689
- US-A1- 2015 032 149
- US-A1- 2018 250 018

## Description

### FIELD

Embodiments of the present techniques relate to a system for treating penetrating wounds, such as stab and gunshot wounds. In particular, the present techniques relate to a system for treating penetrating wounds that comprises a hand-portable actuator and replaceable tamponades that can be coupled to the hand-portable actuator, as well as an expandable tamponade.

### BACKGROUND

Blood loss from penetrating wounds, such as stab and gunshot wounds, can be fatal. A system that enables first responders to reduce the blood loss from such wounds could save many lives.

Background information can be found in: US 2005/0143689 A1, which describes an internal compression tourniquet catheter system and method for controlling haemorrhage from wounds, particularly penetrating wounds; EP 2 896 413 A1, which describes a wound healing system for promoting healing of a wound of a patient including a positive pressure source, a reduced pressure source, and a porous foam positioned in contact with the wound; US 2015/0032149 A1, which describes a portable pneumatic abdominal aortic tourniquet for occlusion of the abdominal descending aorta to restrict blood supply to a non-compressible arterial haemorrhage in or below the inguinal region; WO 2017/181747 A1, which describes a portable automatic tourniquet comprising a cuff; and US 2018/0250018 A1, which describes a device and a method for stopping bleeding at the puncture sites of vessels of patients undergoing an extra corporeal treatment, in particular dialysis.

The present applicant has identified the need for an improved mechanism for reducing blood loss from penetrating wounds.

Background information can also be found in EP 2 896 413 A1 which relates to a wound healing system for promoting healing of a wound of a patient includes a positive pressure source, a reduced pressure source, and a porous foam positioned in contact with the wound. Further background information can be found in CN108969047A which relates to a gynecological hemostatic sac, comprising a uterine cavity, a vaginal capsule, an inflation tube, a sewage tube, a drug delivery tube, an inflatable balloon and a drug injector, the uterine cavity including the inner capsule and the outer surface thereof a drug delivery cavity, the drug delivery cavity is filled with a cotton layer, the drug injector, the drug delivery tube and the drug delivery cavity are sequentially connected, and the inflation balloon, the inflation tube and the inner capsule are sequentially connected.

The present applicant has identified the need for an improved mechanism for reducing blood loss from penetrating wounds.

### SUMMARY

The invention is solely defined in the appended claims.

According to a first approach of the present techniques, there is provided an expandable tamponade for treating penetrating wounds, comprising: an expandable portion comprising: a flexible outer wall (416a); an inner wall (416b); an inner chamber (418) between the flexible outer wall (416a) and the inner wall (416b), wherein the inner chamber (418) is fillable with fluid to expand the expandable portion (109) inside the wound (205); and a channel defined by the inner wall (416b), for receiving a guide probe (404) or inspection probe; a handle mechanism for inserting the expandable portion into a wound, the handle mechanism comprising a housing (804) containing the expandable portion (109) before the expandable portion (109) is inserted into the wound (205); the guide probe (404) for guiding the expandable portion (109) into the wound (205), wherein the guide probe (404) is moveable within the channel, such that a length of the expandable portion (109) within a wound (205) can be varied;a strut for insertion into the wound before the expandable portion and the guide probe (404) to open-up musculature of the wound wherein the housing (804) and strut (412) surround at least part of the expandable portion when the expandable portion is inserted into the wound; and coupling means for coupling the expandable portion to a fluid supply to expand the expandable portion inside the wound.

The expandable tamponade of the present techniques may be suitable for use in any penetrating wound, such as a stab wound, a bullet wound, or any other type of open wound, for example, a wound caused by shrapnel from an explosive device. Thus, it will be understood that the present techniques are not limited to any specific type of wound. The term "penetrating wound" is used interchangeably herein with the terms "wound", "stab wound" and "bullet wound".

The expandable tamponade of the present techniques has multiple advantages. For example, the expandable tamponade is easy to use and compact, making it suitable for first-responders (e.g. paramedics) and soldiers to carry with them and to use. Being able to inflate or expand the expandable portion of the tamponade when it is inside a wound means that the tamponade is configurable/adjustable as the expandable portion can be inflated to the right amount to suit each wound. The strut helps to keep the opening of the wound open, which makes it easier to insert and/or remove the tamponade from the wound.

The expandable portion may be: fixedly connected, at a first end, to the handle; and fixedly connected, at a second end, to a rigid locator tip for guiding the expandable portion through the wound. In some cases, the rigid locator tip may be the first part of the tamponade to be inserted into the wound, and thus may be shaped to ease the tamponade into the wound as well as through the wound. In other cases mentioned below, the strut may be the first part of the tamponade to be inserted into the wound - in this case, the rigid locator tip nevertheless aids the expandable portion to be guided through the wound, as it can push through the tissue and musculature of/around the wound.

In some cases which are not part of the claimed invention but merely examples suitable for understanding the invention, the expandable portion may be a single-walled expandable portion. That is, the expandable portion may comprise a flexible outer wall that defines an inner chamber, wherein the inner chamber is fillable with fluid to expand the expandable portion inside the wound. This means that the expandable portion is only able to receive fluid.

The expandable portion is be a double-walled expandable portion. That is, the expandable portion may comprise: a flexible outer wall; an inner wall; an inner chamber between the flexible outer wall and the inner wall, wherein the inner chamber is fillable with fluid to expand the expandable portion inside the wound; and a channel defined by the inner wall, for receiving a guide probe or inspection probe. This means that the expandable portion is able to receive fluid as well as a probe that advantageously enables the tamponade to be guided into position in the wound and/or enables the wound to be inspected while the tamponade remains in position. The latter is particularly useful because the inspection probe allows medical personnel to understand the nature of the wound and the extent of the damage caused by the foreign object that has pierced the skin and caused the wound (e.g. knife, bullet, shrapnel, etc.) before the tamponade is removed.

In the double-walled case, the inner wall may be fixedly connected at a first end to the handle mechanism, and at a second end to a rigid locator tip for guiding the expandable portion through the wound. That is, the inner wall may be permanently fixed in position such that the channel for a probe is permanently in position in the expandable portion. Alternatively, the inner wall may be fixedly connected at a first end to the handle mechanism, but a second end of the wall is free to move within the inner chamber. In this alternative arrangement, the inner wall and channel may extend into the expandable portion when a probe is inserted into the channel, and at other times, the inner wall may not or may only partially extend into the expandable portion.

The handle mechanism comprises a housing, wherein the housing contains the expandable portion before it is inserted into the wound. That is, the expandable portion may be contained within the housing and only released once the tamponade is to be used (thereby keeping the expandable portion hygienic), or when the strut has been inserted into the wound as explained below.

The expandable portion may be contained within both the housing and the strut of the handle mechanism. In this case, the expandable portion may comprise a rigid locator tip for guiding the expandable portion through the wound, and the rigid locator tip may protrude out through the strut while the expandable portion is contained within the housing. Alternatively, the expandable portion may comprise a rigid locator tip for guiding the expandable portion through the wound, and the rigid locator tip may also be contained within the housing and/or the strut. In this alternative case, the handle mechanism may comprise a seal that seals the strut, thereby ensuring the expandable portion is kept in hygienic conditions. The seal may be removed by a user before use, or the seal may break (by force) when a probe is used to push the expandable portion into a wound.

The expandable portion may be provided in the housing in a rolled, folded or wrapped state.

The housing may comprise a first aperture and a second aperture, wherein the first aperture is for receiving a guide probe for pushing the expandable portion out of the second aperture of the housing, thereby guiding the expandable portion through the wound. As noted above, the force applied by the guide probe may also cause the seal to break (if present).

As noted above, in the double-walled example, a guide probe may be used with the tamponade to guide the expandable portion into a wound. Thus, the handle mechanism for inserting the expandable portion into a wound may itself comprise a guide probe for guiding the expandable portion into the wound.

In some cases, which are not part of the claimed invention but merely examples suitable for understanding the invention, the guide probe may be fixedly provided within the channel, such that the expandable portion is of a fixed length. In this case, the strut may open-up musculature of the wound to enable the expandable portion to be removed from the wound. This can be useful because otherwise the force of the musculature around the expandable tamponade may be such that it is difficult to remove the expandable portion from the wound without considerable force or causing damage.

The guide probe is be moveable within the channel, such that a length of the expandable portion within a wound can be varied. This is particularly useful when the handle mechanism comprises a housing that contains the expandable portion. This is because the length of the guide probe used to push the expandable portion out of the housing can be varied, and thereby, the length of the expandable portion in a wound can be varied. This advantageously makes the tamponade adjustable to suit wounds of different sizes/depths. In this case, the strut may be inserted into the wound, before the expandable portion and guide probe is inserted into a wound, such that the strut opens-up musculature of the wound to enable the expandable portion to be inserted into and removed from the wound.

The guide probe may comprise markings to indicate a depth of the wound into which the expandable portion is inserted. This may help medical personnel to understand the extent of the wound before the tamponade is removed.

In some cases, the guide probe may be a removable component of the handle mechanism, such that the guide probe can be removed after the expandable portion has been inserted into a wound and expanded. This advantageously enables other probes, such as inspection probes, to be inserted into the channel.

The guide probe may be flexible or rigid. A flexible guide probe may be advantageous because wounds may not be completely straight or linear - the flexibility may enable the tamponade to be situated within the wound better.

The guide probe may comprise at least one sensing device. The at least one sensing device may be a sonography or ultrasound device, for example. Such sensors may enable free fluid detection (i.e. detection of internal blood loss) without needing to remove the tamponade and risk destabilising the patient. It will be understood that these are non-limiting example sensing devices.

The expandable portion may comprise a plurality of sections, where each section is independently inflatable. This may advantageously enable the expandable portion to be inflated in a way that suits the shape of the wound.

The tamponade may further comprise a coating on an external surface of at least the expandable portion, wherein the coating comprises any one or more of: a haemostatic agent, an antihemorrhagic agent, and an infection mitigation compound (e.g. silver sulfadiazine). In this way, the surface of the expandable portion may encourage clotting and/or reduce infection. The coating may take the form of granules or particles embedded into or impregnated on the external surface, or may be a gel provided on the external surface.

Additionally or alternatively, the tamponade may comprise at least one layer of gauze around the expandable portion. The at least one layer of gauze may be formed of an absorbent material. The at least one layer of gauze may be impregnated with any one or more of: a haemostatic agent, an antihemorrhagic agent, and an infection mitigation compound.

In a second approach of the present techniques, there is provided a system for treating penetrating wounds comprising: at least one expandable tamponade of the type described herein, and a hand-portable actuator; wherein the at least one expandable tamponade is configured so as to be received within a wound and comprises coupling means to enable the at least one expandable tamponade to be coupled to the hand-portable actuator; and the hand-portable actuator comprises means for expanding the expandable tamponade and coupling means to enable the at least one expandable tamponade to be coupled to the hand-portable actuator so as to enable expansion of the at least one expandable tamponade.

In a third approach of the present techniques, which is not part of the claimed invention but merely an example suitable for understanding the invention, there is provided a system for treating penetrating wounds comprising: at least one expandable tamponade and a hand-portable actuator; wherein the at least one expandable tamponade is configured so as to be received within a wound and comprises coupling means to enable the at least one expandable tamponade to be coupled to the hand-portable actuator; and the hand-portable actuator comprises means for expanding the expandable tamponade and coupling means to enable the at least one expandable tamponade to be coupled to the hand-portable actuator so as to enable expansion of the at least one expandable tamponade and wherein the hand-portable actuator also comprises control means configured to receive an input from one or more pressure sensors indicative of pressure within an expandable tamponade and configured to use the input signals from the pressure sensors to control the means for expanding the expandable tamponade.

The following features apply equally to both the second and third approach.

The hand-portable actuator may be any one of: a bulb air pump; an air- or liquid-filled syringe; an electric actuator; a compressed gas canister; and a mechanical actuator.

The hand-portable actuator may be arranged to supply any one of the following materials into the expandable portion of the expandable tamponade: a material detectable in a medical imaging process; air; a gas; a liquid; a foam; and an expandable foam.

The system may comprise a plurality of different sized and/or shaped expandable tamponades and the coupling means of the hand-portable actuator is configured to enable any of the plurality of different sized and shaped expandable tamponades to be attached to the hand-portable actuator.

The system may comprise means for enabling a user to select which of the plurality of different sized and/or shaped expandable tamponades should be used.

As mentioned above in relation to the first approach, the at least one expandable tamponade may comprise at least one inflatable portion. The inflatable portion may comprise a plurality of sections and each section may be inflated independently of other sections.

The at least one expandable tamponade may comprise an absorptive pad configured to absorb blood external to a wound.

The at least one expandable tamponade may comprise sealing means configured to, at least partially, seal a wound site.

The at least one expandable tamponade may comprise an air release mechanism configured to enable air to be released into the atmosphere.

The hand-portable actuator may comprise control means configured to receive an input from one or more pressure sensors indicative of pressure within an expandable tamponade and configured to use the input signals from the pressure sensors to control the means for expanding the expandable tamponade.

The hand-portable actuator may comprise a power supply configured to provide power to the control means.

The means for expanding the expandable tamponade may comprise one or more pumps.

The hand-portable actuator may comprise one or more valves configured to control air flow from the pump to a tamponade coupled to the hand portable actuator.

The hand-portable actuator may comprise one or more light sources.

The hand-portable actuator may comprise a user interface configured to enable a user to input information to the system and/or enable information to be provided to the user.

The hand-portable actuator may comprise a trigger input configured to enable a user to actuate the means for expanding the expandable tamponade and cause the expansion of an expandable tamponade coupled to the hand-portable actuator.

The system may comprise a case for storing and transporting the hand portable actuator and a plurality of expandable tamponades.

The case may comprise a power source for providing power to the hand portable actuator.

According to a related approach of the present techniques, there is provided a hand portable actuator configured for use in a system as described herein.

### BRIEF DESCRIPTION OF THE DRAWINGS

For a better understanding of various examples of embodiments of the present invention, which is solely defined in the appended claims, reference will now be made by way of example only to the accompanying drawings in which:
Figure 1 illustrates a system for treating penetrating wounds which is not part of the claimed invention but merely an example suitable for understanding the invention;
Figures 2A to 2M illustrate a system for treating penetrating wounds, which is not part of the claimed invention but merely an example suitable for understanding the invention, in use;
Figures 3A to 3C illustrate expandable tamponades that can be used in example embodiments;
Figures 4A and 4B show, respectively, an expandable tamponade which is not part of the claimed invention but merely an example suitable for understanding the invention before and after inflation;
Figures 5A to 5E show different examples of hand-portable actuators;
Figures 6A to 6I show different shapes of the expandable tamponade;
Figures 7A to 7E show an expandable tamponade of variable length;
Figures 8A to 8C are sketches showing how the expandable portion may be housed within a housing;
Figures 9A to 9C show an example arrangement of the expandable tamponade and actuator;
Figures 10A to 10C show an expandable tamponade having at least one layer of gauze;
Figure 11 shows an expandable tamponade comprising multiple inflatable chambers; and
Figures 12A to 12C show an expandable tamponade comprising at least one sensing device.

### DETAILED DESCRIPTION OF THE DRAWINGS

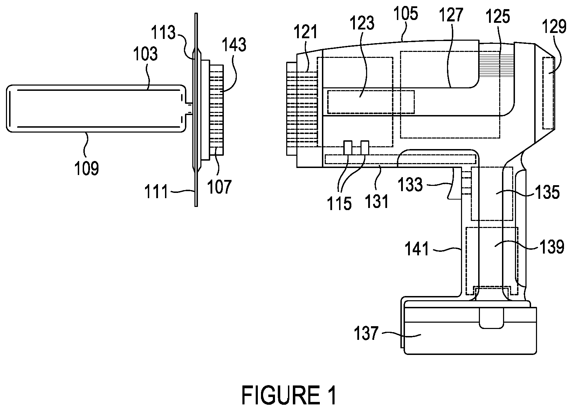

Figure 1 illustrates a system 101 for treating penetrating wounds, such as stab and gunshot wounds. The system 101 comprises two components. The first component is an expandable tamponade 103 that is configured to be received within a wound and the second component is a hand-portable actuator 105 that is configured to be coupled to the expandable tamponade 103 to enable expansion of the tamponade 103. Different sized and/or shaped tamponades can be used for different wounds.

The expandable tamponade 103 is configured to be received within a wound. The expandable tamponade 103 comprises an inflatable portion 109 that has an elongate shape. The elongate shape can be sized and shaped so as to enable the inflatable portion 109 to be inserted into a wound.

The inflatable portion 109 can comprise a single section as shown in Figure 1. In other examples the inflatable portion 109 can comprise a plurality of sections. Each section can be sealed from the other sections so as to enable the different sections to be inflated independently. This can enable different sized and shaped inflated tamponades to be provided.

The inflatable portion 109 can be formed from any suitable material. In some examples the inflatable portion 109 can be formed from a biocompatible material such as medical grade silicone.

The expandable tamponade 103 also comprises coupling means 107. The coupling means 107 can comprise any means that can enable the expandable tamponade 103 to be coupled to the hand-portable actuator 105. In the example shown in Figure 1 the coupling means 107 comprises a connector that is sized and shaped so as to be received within a corresponding receiving means within the hand-portable actuator 105.

The coupling means 107 can be configured to enable a user to attach the expandable tamponade 103 to the hand-portable actuator 105 for use of the system 101. The coupling means 107 can be releasable coupling means 107 so that the user can separate the hand-portable actuator 105 from the expandable tamponade 103 once the expandable tamponade 103 has been expanded or at any other suitable point.

The coupling means 107 can be configured so that the expandable tamponade 103 can be easily attached to, and detached from, the hand-portable actuator 105 by the user as needed. The user does not need any specialist tools to attach and/or detach the expandable tamponade 103 to the hand-portable actuator 105.

The coupling means 107 can comprise one or more guide features to help a user connect the hand-portable actuator 105 in the correct orientation. The guide features could comprise grooves or projections that fit into the hand-portable actuator 105 in a particular orientation. In some examples the guide features could comprise a sign or other indication that informs the user of the correct orientation.

In the example of Figure 1 the expandable tamponade 103 comprises an absorptive pad 111. The absorptive pad 111 can be configured to absorb blood that is external to the wound. The absorptive pad 111 can comprise gauze or any other suitable material that can be used to absorb blood.

The absorptive pad 111 is provided at the open end of the inflatable portion 109. The absorptive pad 111 is configured so that when the expandable tamponade 103 is in use the absorptive pad 111 remains on the outside of the wound, or at least partially on the outside of the wound. In the example shown in Figure1 the absorptive pad 111 can be provided so that it surrounds, or at least partially surrounds, the coupling means 107.

In the example of Figure 1 the expandable tamponade 103 also comprises sealing means 113. The sealing means are configured to, at least partially seal the wound. This can keep the wound site clean and can also help to secure the expandable tamponade 103 in place and reduce blood loss from the wound.

The sealing means 113 can be provided around the edges of the absorptive pad. The sealing means 113 can be configured to form a seal against the patient's skin.

In some examples the sealing means 113 can also comprise an attachment bandage. The attachment bandage can be coupled to the outside of the sealing means 113 and can be used to secure the inserted expandable tamponade 103 in place within the wound. The attachment bandage could comprise a roll of bandage or gauze or any other suitable means.

It is to be appreciated that the expandable tamponades 103 could also comprise additional features and components that are not shown in Figure 1. For instance, the expandable tamponades 103 could comprise an air release mechanism 143. This could comprise a valve or any other suitable means that, once actuated, causes the air within the inflatable portion 109 to be released into the atmosphere. This can allow the expandable tamponade 103 to be removed from the wound when it is no longer needed. The air release mechanism 143 can be configured to allow a portion of the air to be released from the inflatable portion 109.

In some examples the expandable tamponade 103 could comprise one or more pressure relief valves. The pressure relief valves could comprise valves that automatically vent gas from the inflatable portion 109 if the pressure within the inflatable portion 109 exceeds a predetermined threshold. The threshold value of the pressure may be dependent upon a number of different factors. The threshold pressure could be above 220mmHg or any other suitable value.

In some examples the expandable tamponade 103 could comprise one or more probe vents. The vents could comprise means for inserting one or more probes into the expandable tamponade 103 without relieving the pressure within the expandable tamponade 103. The probes can be used to obtain images or other information about the internal wound that can be used to treat the patient.

In some examples the expandable tamponade 103 could comprise one or more thermal sensors. The thermal sensors can be distributed around the expandable tamponade 103 to allow for thermal imaging of the wound or portions of the wound. The thermal sensors can provide an indicate of the temperature of different portions of the wound. This can provide information about which regions of the would have blood clotting and which regions have continued blood flow.

The hand-portable actuator 105 is sized and shaped so that it can be easily carried by a user. The hand-portable actuator 105 is shaped so that a single user can hold the hand-portable actuator 105 while it is in use. The hand-portable actuator 105 can be sized and shaped so that a user can hold the hand-portable actuator 105 in a single hand while they are using the hand-portable actuator 105.

In the example shown in Figure 1 the hand-portable actuator 105 has a handle portion 141. The handle portion 141 is sized and shaped so that a user can grip it with their hand while the hand-portable actuator 105 is in use. Other shapes and arrangements of the hand-portable actuator 105 could be used in other examples of the invention.

The hand-portable actuator 105 comprises coupling means 121. The coupling means 121 can comprise any means that can enable the hand-portable actuator 105 to be coupled to the different expandable tamponades 103 provided with the system 101. In the example shown in Figure 1 the coupling means 121 comprises a receiving means that is sized and shaped so as to receive a corresponding connector from an expandable tamponade 103.

The coupling means 121 can be configured to enable a user to attach the expandable tamponade 103 to the hand-portable actuator 105 for use of the system 101. The coupling means 121 can be releasable coupling means 107 so that the user can separate the hand-portable actuator 105 from the expandable tamponade 103 once the expandable tamponade 103 has been inflated or at any other suitable point.

The coupling means 121 can be configured so that the expandable tamponade 103 can be easily attached to, and detached from the hand-portable actuator 105 by the user as needed. The user does not need any specialist tools to attach and/or detach the expandable tamponade 103 to the hand-portable actuator 105.

In the example shown in Figure 1 the coupling means 121 are configured so that the hand-portable actuator 105 can be coupled to the expandable tamponade 103 without any intervening components. In other examples one or more intervening components could be provided. For example, a flexible hose or other means could be attached to the coupling means 121 so that the hand-portable actuator 105 can be coupled to the expandable tamponade 103 via the flexible hose. The flexible hose can allow for relative movement of the hand-portable actuator when it is connected to the expandable tamponade 103. The flexible hose can also provide a conduit for air, or other gases, from the hand-portable actuator 105 to the expandable tamponade 103.

The hand-portable actuator 105 also comprises means for expanding the expandable tamponade 103. In the example of Figure 1 the means for expanding the expandable tamponade comprises a pump 127. Other means could be used in other examples of the invention.

The pump 127 can comprise an air compressor 125 and a valve 123. The air compressor 125 can be configured to draw in air from the environment and direct the air through the valve 123 towards the expandable tamponade 103. This can enable air from the pump 127 to be used to inflate the inflatable portion 109 of the expandable tamponade 103 when the expandable tamponade 109 is attached to the hand-portable actuator 105. The respective coupling means 107, 121 of the expandable tamponade 103 and the hand-portable actuator 105 can be configured to allow air from the pump 127 to flow into the inflatable portion 109 of the expandable tamponade 103. The valve 123 can prevent air flowing back from the expandable tamponade 103 into the hand-portable actuator 105.

In some examples the hand-portable actuator 105 can also comprise a vacuum pump. The vacuum pump can be used to remove air or other gases from the inside of the inflatable portion 109 as needed. In some examples the vacuum pump can be used automatically during the inflation of the inflatable portion 109. This helps to regulate pressure within the inflatable portion 109. In some examples the vacuum pump can also allow a user to remove air or other gases from within the inflatable portion 109 if the pressure exceeds a given threshold.

The hand-portable actuator 105 can also comprise one or more sensors 115. The sensors 115 can comprise pressure sensors or any other suitable types of sensors. The pressure sensors 115 can be provided in any suitable position within the hand-portable actuator 105. For example, they can be provided within a gas inlet and/or a gas outlet. The pressure sensors 115 can be configured to enable the pressure within the inflatable portion 109 of the expandable tamponade 103 to be determined. The pressure sensors 115 can be configured to detect when the pressure applied by the inflatable portion 109 to the wound as the inflatable portion 109 is inflated reaches a threshold value. The threshold value could be around 180mmHg or any other suitable value. The pressure sensors 115 can be configured to provide an output when it is detected that threshold value has been reached. The output signal can be provided from the expandable tamponade 103 to the hand-portable actuator 105. In other examples the pressure sensors 115 could be provided within the expandable tamponade 103. In such examples the expandable tamponade 103 can comprise means for providing an indication of the pressure to the hand-portable actuator 105.

The hand-portable actuator 105 also comprises a user interface 129. The user interface 129 can comprise any means that enables information to be output to a user of the hand-portable actuator 105. The hand-portable actuator 105 can comprise a display, a plurality of LEDs (light emitting diodes), one or more loudspeakers or any other suitable means for providing information to a user.

In some examples the user interface 129 can also comprise input means that can enable a user to input information to the hand-portable actuator 105. For example, it can enable a user to make one or more inputs indicating the type of wound or any other suitable information.

In the example of Figure 1 the user interface 129 is positioned on the hand-portable actuator 105 so that the user can view the user interface 129 while the hand-portable actuator 105 is in use. In the example of Figure 1 the user interface 129 is provided on a rear face of the hand-portable actuator 105.

In some examples the user interface 129 can also comprise one or more LEDs that can be configured to provide information to a user of the hand-portable actuator 105. The LEDs can be located in any suitable positions on the surface of the hand-portable actuator 105. For instance, some LEDs could be provided around a display unit. The LEDs could provide green light if the system 101 is being used correctly or red light if the system is being used incorrectly. For example, the LEDs could provide red light if the pressure within the expandable portion 109 has exceeded a given threshold or if the expandable tamponade 103 has not been inserted correctly or if any other problems are detected.

The hand-portable actuator 105 also comprises control means 131. The control means 131 can comprise control circuitry such as a processing chip or any other suitable means. The control means 131 can be configured to control one or more components of the hand-portable actuator 105. In some examples the control means 131 can be configured to control the pump 127, the user interface 129 and/or any other suitable components.

In some examples of the disclosure the control means 131 can be configured to receive one or more input signals from the one or more pressure sensors 115. This input from the pressure sensors 115 can be used by the control means 131 to determine if the inflatable portion 109 has been fully inflated or if the inflatable portion 109 has been sufficiently inflated. This can be used to control the pump 127 and control the air being provided to the inflatable portion 109. For instance, if it is determined that the inflatable portion 109 is fully inflated then the control means 131 can deactivate the pump 127 and prevent any further inflation of the inflatable portion 109.

The hand-portable actuator 105 also comprises a trigger input 133 and a trigger sensor 135. The user of the hand-portable actuator 105 can use the trigger input 133 to activate the pump 127 once the expandable tamponade 103 has been connected to the hand-portable actuator 105 and the inflatable portion 109 of the expandable tamponade 103 has been inserted into the wound. The trigger sensor 135 can detect when the trigger input 133 has been actuated and provide a control input to the pump 127.

The hand-portable actuator 105 also comprises a power source 137. The power source 137 can comprise a rechargeable battery or any other suitable means. The power source 137 can be configured to provide power to the components of the hand-portable actuator 105 as needed. For example, the power source 137 can be configured to provide power to the pump 127, the control means 131, the user interface 129 and any other suitable components. The power source 137 can be coupled to a power converter 139 that can be configured to convert the power from the power source 137 to a suitable format for use by the components of the hand-portable actuator 105.

It is to be appreciated that the hand-portable actuator 105 can comprise components that are not shown in Figure 1. For instance, in some examples the hand-portable actuator 105 can comprise one or more light sources. The light sources could be configured to illuminate the area around the wound when the system 101 is in use or to provide any other suitable illumination.

Only one expandable tamponade 103 is shown in Figure 1. However, it is to be appreciated that the system 101 could comprise a plurality of different expandable tamponades 103. The different expandable tamponades 103 could have different sized and/or shaped inflatable portions 109. This can make the different expandable tamponades 103 suitable for use in different types of wounds. For instance, the larger expandable tamponades 103 could be suitable for use in wounds applied to the abdomen while the smaller expandable tamponades 103 could be suitable for use in wounds applied to the armpit or groin areas.

Each of the different expandable tamponades 103 can be provided with a coupling means 107 that enables it to be attached to the hand-portable actuator 105. The coupling means 107 on each of the different expandable tamponades 103 can be the same so that the same hand-portable actuator 105 can be used with a plurality of different expandable tamponades 103 as needed.

It is to be appreciated that the system 101 could comprise additional components that are not shown in Figure 1. For instance, in some examples the system 101 could comprise a case for storing the hand-portable actuator 105 and a plurality of expandable tamponades 103. The case could be sized and shaped so that it can be easily carried by a user. The case could be sized and shaped so that it can fit inside a vehicle such as a police car. The case could also comprise additional equipment that can be used when using the system 101. For example, the case could comprise spare batteries, charging means, personal protective equipment, scissors for removing clothing from a patient, wound dressings, instructions for use or any other suitable equipment.

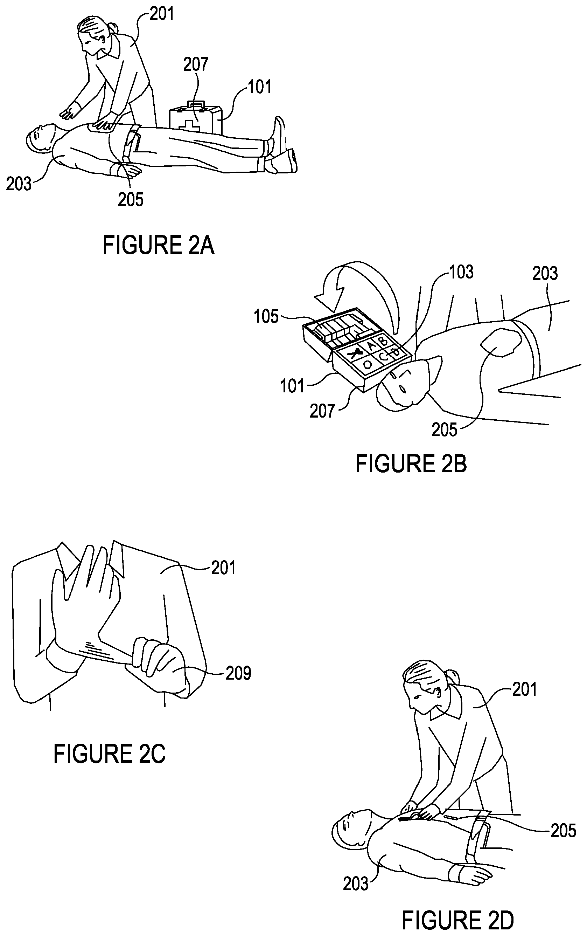

Figures 2A to 2M show an example system 101, such as the system of Figure 1, in use.

In Figure 2A a user 201 of the system 101 has found a patient 203 who has suffered a wound 205. The wound 205 has been inflicted on the abdomen of the patient 203. The user 101 could be a first responder such as a paramedic, a police officer or any other suitable first aider.

The user 201 has brought the system 101 with them. The system 101 is provided within the case 207. The case 207 can be easily carried by the user 201 and contains all the parts of the system 101 that the user 201 will need.

In Figure 2B the user 201 has opened the case 207 containing the system 101. The hand-portable actuator 105 is provided on a first side of the case 201 and plurality of expandable tamponades 103 are provided on another side of the case 207. The expandable tamponades 103 are provided in individual, aseptically sealed packaging. Each part of the system 101 can be clearly labelled within the case 207 so that the user 201 can easily identify and find the items that they need.

In this example the different expandable tamponades 103 are provided in different compartments within the case 207. The case 207 can be configured so that different sized and/or shape expandable tamponades 103 are provided within the different compartments. Each of the different compartments can be labelled so that a user 201 can find the correct expandable tamponades 103 when needed. In this example four different compartments are provided. The different compartments are labelled A, B, C and D. Other numbers of compartments can be provided in other examples of the disclosure. Other means for labelling or identifying the different expandable tamponades 103 can also be used in other examples of the invention.

In Figure 2C the user 201 puts on personal protective equipment 209. In this example the personal protective equipment 209 comprises gloves. Other types of personal protective equipment 209 could be used in other examples. For instance, the personal protective equipment 209 could comprise aprons, facemasks or any other suitable equipment.

The personal protective equipment 209 can be provided within the case 207 so that the personal protective equipment 209 is available whenever the system 101 is used.

In Figure 2D the user 201 clears the wound 205. The user 201 can remove any obstructions from the wound 205 to enable the expandable tamponade 103 to be inserted into the wound 205. The equipment for cleaning the wound 205 can be provided within the case 207.

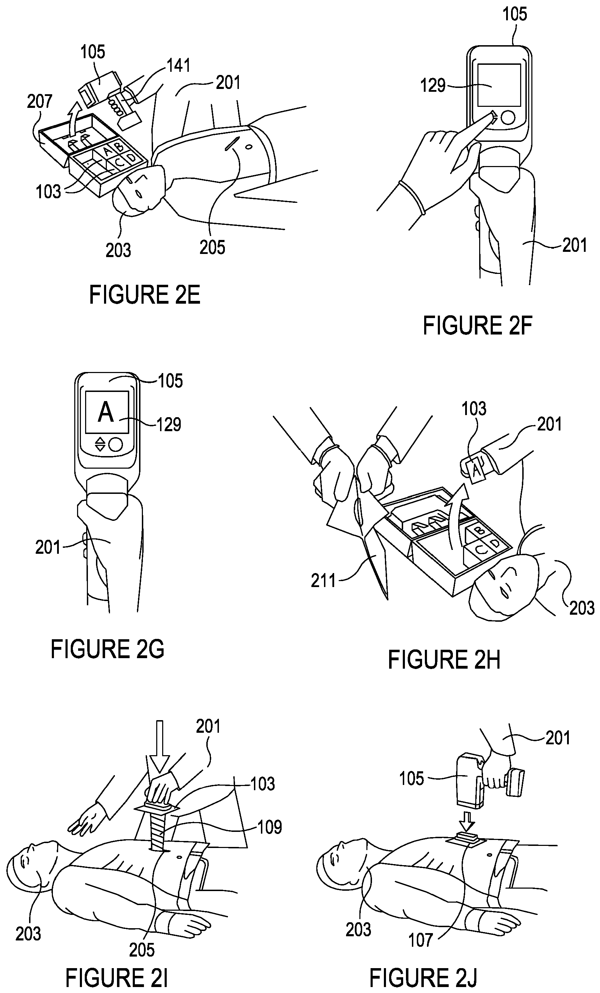

In Figure 2E the user 201 removes the hand-portable actuator 105 from the case 207. The user 201 holds the hand-portable actuator 105 by the handle portion 141.

In Figure 2F the user 201 uses the user interface 129 of the hand-portable actuator 105 to indicate the type of wound 205 that is to be treated. For instance, the user 201 can indicate the location of the wound 105, information relating to the patient and/or any other suitable information. In this example the user 201 could indicate that the wound 205 has been inflicted on the torso of the patient 203 and/or any other suitable information.

The user 201 can hold the hand-portable actuator 105 in one hand while they use their other hand to input the required information.

Once this information has been input the control means 131 will determine which of the plurality of available expandable tamponades 103 should be used to treat this wound 205. The selection of the expandable tamponade 103 that is to be used can be based on the expected size of the wound 105 and/or any other suitable information.

In Figure 2G the control means 131 controls the user interface 129 to provide an output to the user 201 indicating the expandable tamponade 103 that is to be used. In this example a notification is provided on the display of the user interface 129 that indicates the expandable tamponade 103 that is to be used. Other means for indicating the expandable tamponade 103 that is to be used could be implemented in other examples of the invention. For instance, one of more LEDs could be illuminated to indicate the expandable tamponade 103 or an audio output could be provided or any other suitable means.

In the example of Figure 2G the user interface 129 indicates that the expandable tamponade 103 labelled A is to be used. The user 201 can the retrieve the corresponding expandable tamponade 103 from the compartment labelled A in the case 207 as shown in Figure 2H. The user 201 can then remove the expandable tamponade 103 from any packaging 211. The packaging 211 can be sterile packaging or any other suitable type of packaging 211.

In Figure 2I the user 201 inserts the expandable tamponade 103 into the wound 205. The expandable tamponade 103 is in a non-expanded state when it is inserted into the wound 205.

The expandable tamponade 103 can be inserted into the wound 205 so that the inflatable portion 109 is fully inserted into the wound 205.

When system 101 is in use the inflatable portion 109 is inserted into the wound to enable the pressure from the inflatable portion to reduce bleeding within the wound. The inflatable portion 109 can be fully inserted into the wound so that it is not visible to the user 201. The inflatable portion 109 can be fully inserted into the wound 205 so that the absorptive pad 111 and/or the sealing means 113 contact the skin of the patient 203.

When the inflatable portion 109 has been inserted in to the wound 205 the coupling means 107 of the expandable tamponade 103 is provided extending out of the wound 205. The user 201 can then attach the coupling means 121 of the hand-portable actuator 105 to the coupling means 107 of the expandable tamponade 103 as shown in Figure 2J.

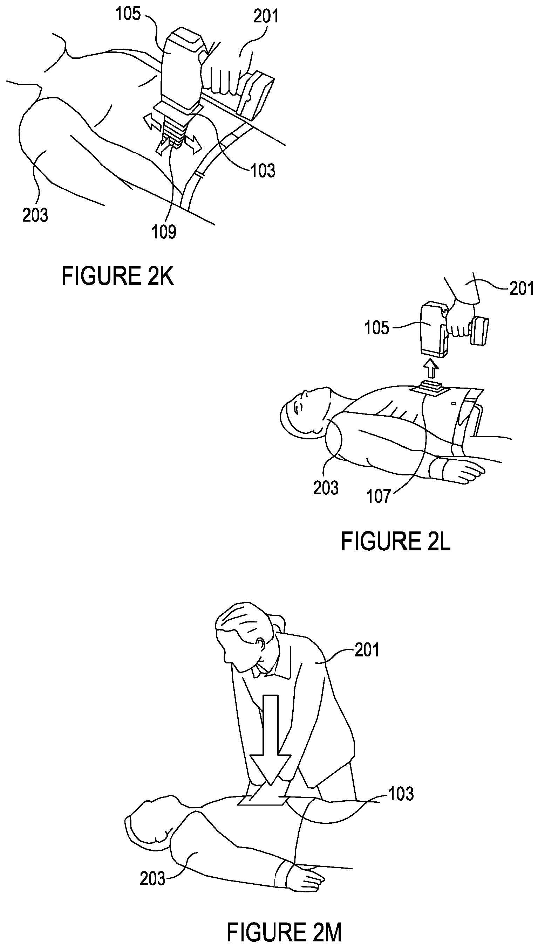

Once the hand-portable actuator 105 is coupled to the expandable tamponade 103 the user 201 actuates the trigger input 133. The trigger sensor 135 detects that the trigger input 133 has been actuated and activates the pump 127. In response to the actuation of the trigger sensor 135 the pump 127 drives air though the respective coupling means 121, 107 and into the expandable tamponade 103. This causes inflation of the inflatable portion 109. The inflation causes the inflatable portion 109 to expand as indicated by the arrows in Figure 2K.

The user 201 can hold the hand-portable actuator 105 by the handle portion 141 while the hand-portable actuator 105 is being used to inflate the expandable tamponade 103. This can enable the hand-portable actuator 105 to be held in a steady position relative to the expandable tamponade 103. This can also enable the user 201 to apply pressure to the wound 205 which can also help to suppress any bleeding.

As the inflatable portion 109 expands this applies pressure to the internal parts of the wound 205. This pressure can help to reduce the bleeding.

Once the inflation of the inflatable portion 109 has been completed the hand-portable actuator 105 can be removed from the expandable tamponade 103. The coupling means 121 of the hand-portable actuator 105 can be detached from the coupling means 107 of the expandable tamponade 103 as shown in Figure 2L. When the hand-portable actuator 105 is removed from the expandable tamponade 103 the inflatable portion 109 of the expandable tamponade 103 remains inside the wound 205.

The control means of the hand-portable actuator 105 can use signals obtained from the expandable tamponade 103 to determine when the expandable tamponade 103 has been sufficiently inflated. For example, one or more sensors 115 such as pressure sensors can be used to detect level of inflation of the inflatable portion 109. This information can be provided to the control means 131 of the hand-portable actuator 105 and used to control one or more components of the hand-portable actuator 105.

In some examples when the control means 131 detects that the inflatable portion 109 has been sufficiently inflated it can control the hand-portable actuator 105 to provide an output to the user 201. The output could comprise a notification displayed on a display of the user interface 129, the illumination of one or more LEDs, an audio output or any other suitable output. The output can indicate to the user 201 that the inflatable portion 109 has been sufficiently inflated. Once the user 201 has received the notification the user 201 can release the trigger input 133 which will cause the pump 127 to be deactivated and prevent any more air being provided to the inflatable portion 109.

In other examples when the control means 131 detects that the inflatable portion 109 has been sufficiently inflated it can control the hand-portable actuator 105 to deactivate the pump 127 without any further input from the user 201. The user 201 could then be provided with a notification that the hand-portable actuator 105 can be detached from the expandable tamponade 103.

Once the hand-portable actuator 105 has been removed the user 201 applies a downward force to the expandable tamponade 103 by pushing down on the connecting means 107 or any other part that remains external to the patient 203 as shown in Figure 2M. The user 201 can continue applying this force to the expandable tamponade 103 until further medical assistance is available.

Once further medical assistance is available the expandable tamponade 103 can be removed from the wound 205. The expandable tamponade 103 can be deflated, or at least partially deflated, before it is removed from the wound 205. The air release mechanism of the expandable tamponade 103 can be actuated to enable the air to be released from the inflatable portion 103 into the environment.

The components of the system 101 that have been used can be replaced within the case 207 as needed. Once the expandable tamponade 103 has been used it should be discarded. However, another replacement expandable tamponade 103 can be added to the case 207 in preparation for the next time that the system 101 is used. The hand-portable actuator 105 can be a multi-use actuator and can be used on a plurality of different occasions. In some examples the power source 137 of the hand-portable actuator 105 may need to be recharged after use of the hand-portable actuator 105. In some examples one or more additional or replacement power sources can be provided within the case 207. In some examples a larger power source within the case 207 can be used to recharge the power sources 137 and/or to power the hand-portable actuator 105 directly.

Embodiments of the invention therefore provide for a system for treating wounds. The system 101 is simple and easy for a user 201 to use. The user does not need to be a trained medical professional. The system 201 can be configured to instruct the user 201 how to use it. Also, the pressure that is applied by the inflatable portion 109 can be controlled by the pressure sensors 115 and the control means 131 of the hand-portable actuator 105. This means that the user 201 does not need to make any judgements about the pressure that needs to be applied.

In embodiments of the invention the air is provided to the inflatable portion 109 via a pump 127 or any other suitable mechanized means. This means that the user 201 does not need to manually pump air into the inflatable portion 109. This can make the system 101 more convenient to use and can also make the system 101 faster to use.

The system 101 can be configured to treat a plurality of different sized and shaped wounds 205 by having a plurality of different sized and/or shaped expandable tamponades 103 available. This can provide improved suppression of bleeding for the different types of wound 205 and/or different types of patient.

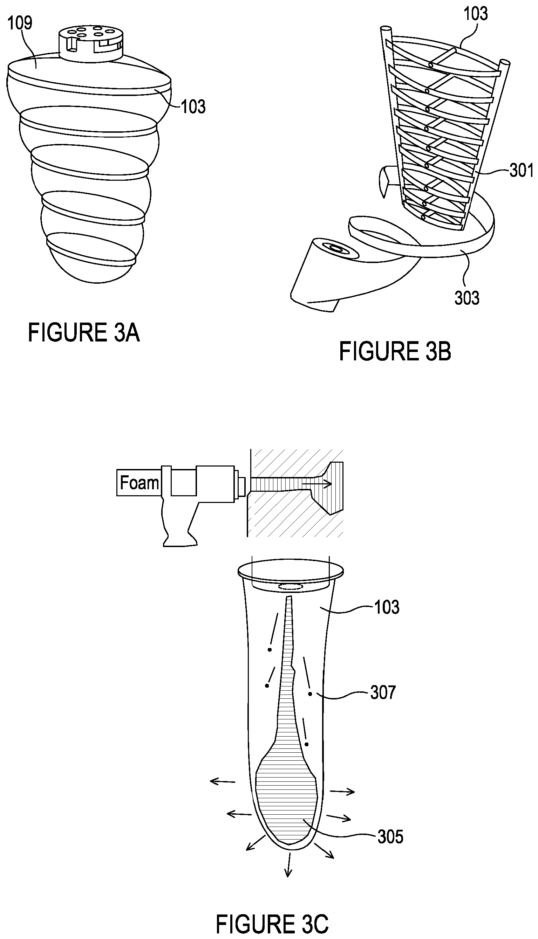

Figures 3A to 3C show example variations of the expandable tamponade 103 that can be used in some examples of the disclosure.

Figure 3A shows an expandable tamponade 103 comprising an inflatable portion 109. The inflatable portion 109 is configured to be inflated by air supplied from a pump 127 in the hand-portable actuator 105.

Figure. 3B shows an expandable tamponade 103 comprising an expandable skeleton 301 surrounded by a gauze wrapping 303. The expandable skeleton 301 can comprise sprung steal or any other suitable material. The expandable skeleton 301 can be expanded in response to an actuating force applied by the hand-portable actuator 105 or by any other suitable means.

Figure 3C shows an expandable tamponade 103 comprising an expandable portion 307 that is configured to be filled with an expanding foam 305 or any other suitable material. The expanding foam 305 can be provided to the expandable tamponade 103 from the hand-portable actuator 105 or from any other suitable means.

It is to be appreciated that variations can be made to the examples shown in Figures 1 to 3C. For instance, in the examples shown the hand-portable actuator 105 comprises a trigger input 133 that allows for some manual control of the inflation of the inflatable portion 109. In other examples the trigger input 133 is not provided and the inflation of the inflatable portion 109 could be controlled automatically without any specific user input.

In other examples the pump 127 could be replaced with a different means of inflating or expanding the expandable tamponades 103. For example, a liquid could be used instead of air or a manual or hand pump could be provided.

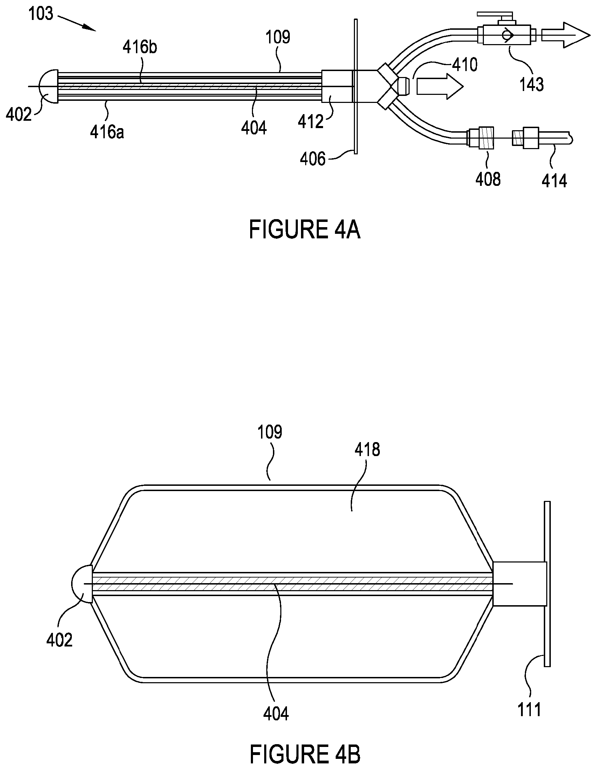

The expandable tamponade is now described in more detail with reference to Figures 4A to 17C.

Figures 4A and 4B show, respectively, the expandable tamponade before and after inflation. As mentioned above, the present techniques provide an expandable tamponade 103 1for treating penetrating wounds such as stab and gunshot wounds. The expandable 103 tamponade comprises an expandable portion 109; a handle mechanism 406 for inserting the expandable portion 109 into a wound, the handle mechanism 406 comprising a strut 412 for insertion into the wound to open-up musculature of the wound; and coupling means 408 for coupling the expandable portion to a fluid supply to expand the expandable portion inside the wound.

The expandable portion 109 of the expandable tamponade 103 may be: fixedly connected, at a first end, to handle 406; and fixedly connected, at a second end, to a rigid locator tip 402 for guiding the expandable portion 109 through the wound. In some cases, the rigid locator tip 402 may be the first part of the tamponade 103 to be inserted into the wound, and thus may be shaped to ease the tamponade into the wound as well as through the wound. In other cases mentioned below, the strut 412 may be the first part of the tamponade to be inserted into the wound - in this case, the rigid locator tip nevertheless aids the expandable portion to be guided through the wound, as it can push through the tissue and musculature of/around the wound.

The expandable portion 109 may be a double-walled expandable portion. That is, the expandable portion may comprise: a flexible outer wall 416a; an inner wall 416b; an inner chamber 418 between the flexible outer wall and the inner wall, wherein the inner chamber is fillable with fluid to expand the expandable portion 109 inside the wound; and a channel defined by the inner wall 416b, for receiving a guide probe 404 or inspection probe. This means that the expandable portion is able to receive fluid as well as a probe that advantageously enables the tamponade to be guided into position in the wound and/or enables the wound to be inspected while the tamponade remains in position. The latter is particularly useful because the inspection probe allows medical personnel to understand the nature of the wound and the extent of the damage caused by the foreign object that has pierced the skin and caused the wound (e.g. knife, bullet, shrapnel, etc.) before the tamponade is removed.

The probe 404 may be removable or non-removable. This is described in more detail below.

The guide probe 404 may be flexible or rigid. A flexible guide probe 404 may be advantageous because wounds may not be completely straight or linear - the flexibility may enable the tamponade to be situated within the wound better. A flexible probe 404 may help navigate the wound track. A flexible probe 404 may be stiffer than the material used for the expandable portion 109 of the expandable tamponade 103 but flexible enough to conform to the shape of the wound track, which is helpful for wounds that are not perpendicular to the surface of the body part where the wound exists. A rigid guide probe 404 gives the expandable tamponade 103 stiffness, so that the expandable portion can be easily inserted into a wound. on.

The expandable portion 109 of the expandable tamponade may be made from a non-elastic material that can be inflated. The expandable portion 109 does then not have any inherent stiffness and the guide probe 404 allows for the expandable portion 109 to be inserted sufficiently deep into the wound.

The locator tip 402 caps the expandable portion 109 of the expandable tamponade and connects and seals different parts of the expandable tamponade together. When inserting the expandable tamponade into a wound tract, the locator tip 402 pushes into the wound tract and opens it up so that the expandable portion 109 can be successfully inserted. The (dwelling) locator tip 402 may be made from a stiffer material than the expandable tamponade and may be shaped in various ways. For example, the dwelling locator tip 402 may have a hemispherical shape or a rounded shape.

In the example shown in Figure 4A, the coupling means is a barb tube connector 408 that connects via tubing 414 to the hand-portable actuator 105. Figure 4A also shows a pressure relief valve 410 that may automatically vent excess fluid if the pressure within the expandable tamponade 109 reaches a pre-set threshold value, and the air release mechanism 143.

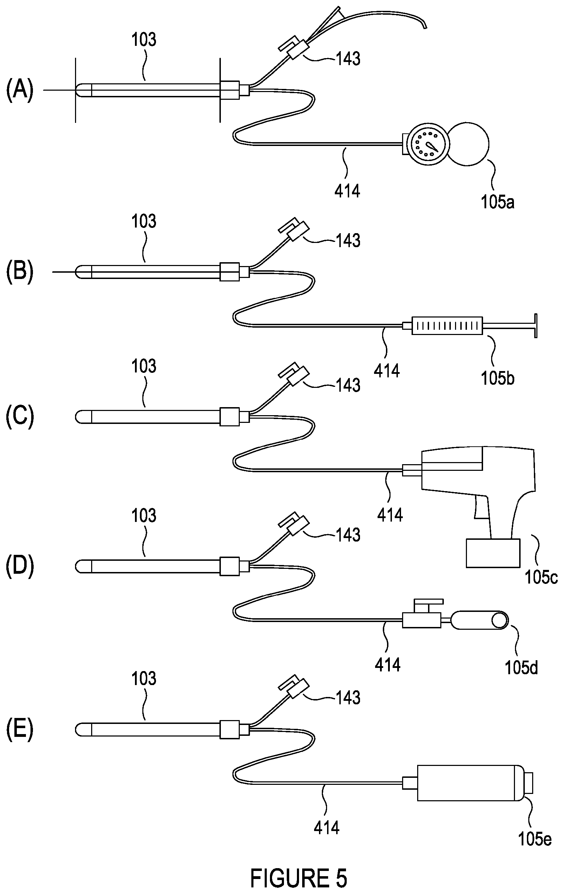

Figures 5A to 5E show different examples of hand-portable actuators 105 that are, for example, connectable to the expandable tamponade 103 via tubing 414. Generally speaking, the hand-portable actuator may be any one of: a bulb air pump; an air- or liquid-filled syringe; an electric actuator; a compressed gas canister; and a mechanical actuator. It will be understood this is a non-limiting list of example hand-portable actuators.

When using a standard barb tube connector of the type shown in Figure 4A, any available actuator device that can be connected to the standard connector may be used, which is advantageous when quickly wanting to treat a deep wound. The tubing may be standard tubing as used in medical settings, or it may be a different type of tubing. Figure 5A shows using a bulb air pump 105a, as is widely available in medical settings, as a hand-portable actuator. Figure 5B shows inflating the expandable tamponade using a syringe 105b filled with air or another fluid. The fluid may be saline or any other suitable fluid. As shown in Figure 3C, the expandable tamponade may also be inflated using expanding foam 305 that is injected into the expandable tamponade 103 by any of the hand-portable actuators 105a to 105e shown in Figures 5A to 5E. More generally, the hand-portable actuator may be arranged to supply any one of the following materials into the expandable portion of the expandable tamponade: a material detectable in a medical imaging process; air; a gas; a liquid; a foam; and an expandable foam. It will be understood this is a non-limiting list of example materials that may be used to expand the expandable portion of the tamponade.

Introducing air into the body can be dangerous, which is why it is important that the expandable tamponade 103 is rupture-resistant, even when in contact with potential shrapnel or other sharp objects that may remain in a wound after it has been inflicted. Therefore, it may be preferable to use an actuator device that inflates the inflatable portion 109 of the expandable tamponade with 103 with a fluid that is not harmful, even in case the expandable tamponade 103 is punctured, such as saline.

Figure 5C shows inflating the inflatable portion 109 of the expandable tamponade 103 using an electric hand-portable actuator of the type as shown in Figure 1. This type of hand-portable actuator 105c may be carried by first responders or it may be used by surgeons or other medical staff when a patient has been transported to hospital. Using an electric hand-portable actuator in a medical setting would make it easier for the hand-portable actuator 105c to remain sterile. Preventing the transfer of, for example, blood from one patient to another through the hand-portable actuator 105c should be avoided. This is why the use of disposable hand-portable actuators, of which examples are shown in Figures 5A to 5E, may be preferrable in a non-sterile setting outside of a hospital.

Figure 5D shows inflating the inflatable portion 109 of the expandable tamponade 103 using a compressed gas canister 105d. Figure 5E shows a mechanical hand-portable actuator 105e that is used to inflate the inflatable portion 109 of the expandable tamponade 103.

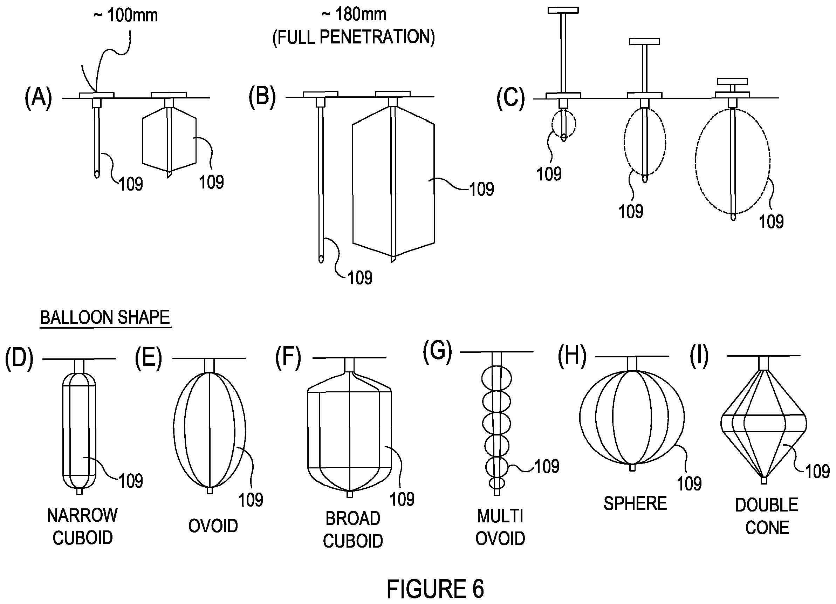

Figures 6A to 6I show the different shapes that the expandable tamponade 103 may have. The size, shape and length of the expandable portion 109 of the expandable tamponade 103 may vary. Figure 6A shows the expandable portion 109 in a non-expanded state (left) and expanded state (right). In this case, the expandable portion 109 may have a length of 100mm. In contrast, Figure 6B shows an expandable tamponade that may have a length of 180mm. Different lengths of tamponade may be advantageous because wounds may vary in depth, and thus an appropriate tamponade may be used for a wound.

Figure 6C shows an expandable tamponade that has an expandable portion 109 of variable length. In this case, as described in more detail below, a guide probe may be used to vary a length of the expandable portion within a wound. This advantageously makes the tamponade adjustable to suit wounds of different sizes/depths. The strut may be inserted into the wound, before the expandable portion and guide probe is inserted into a wound, such that the strut opens-up musculature of the wound to enable the expandable portion to be inserted into and removed from the wound.

Figures 6A to 6I show expandable portions 109 having different shapes. Tamponades having particular shapes may be more suitable to certain wounds. That is, the type of tamponade used may depend on the location, type and/or size of the wound. The expandable tamponade 103 of the present techniques may be used to stem the bleeding from any type of wound. This may, for example, be a stab wound, a gunshot wound or a wound caused by shrapnel.

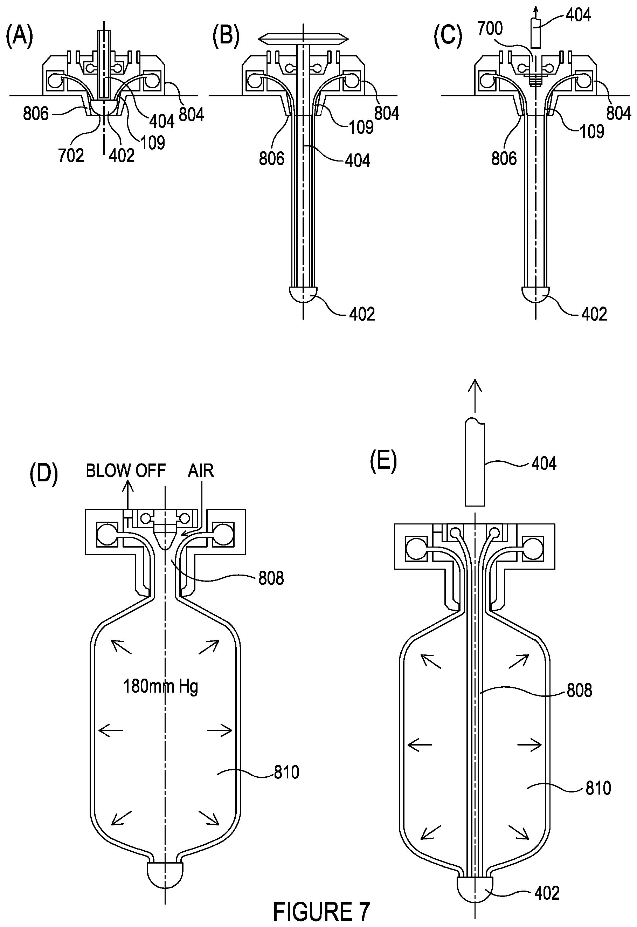

As mentioned above, it is advantageous to have a variable length expandable tamponade. Figures 7A to 7E show an expandable tamponade 103 of variable length. The handle mechanism of the expandable tamponade may comprise a housing 804, wherein the housing 804 contains the expandable portion 209 before it is inserted into a wound. That is, the expandable portion 209 may be contained within the housing 804 and only released once the tamponade is to be used (thereby keeping the expandable portion 109 hygienic), or when the strut 806 has been inserted into the wound as explained below.

The expandable portion may be contained within both the housing 804 and the strut 806 of the handle mechanism. The expandable portion 109 may comprise a rigid locator tip 402 for guiding the expandable portion through the wound, and the rigid locator tip may protrude out through the strut while the expandable portion is contained within the housing. Alternatively, as shown in Figure 7A, the rigid locator tip for guiding the expandable portion through the wound may also be contained within the housing 804 and/or the strut 806. In this alternative case, the handle mechanism may comprise a seal (not shown) that seals the strut, thereby ensuring the expandable portion is kept in hygienic conditions. The seal may be removed by a user before use, or the seal may break (by force) when a probe is used to push the expandable portion into a wound.

As shown in Figure 7A and 7C, the housing 804 may comprise a first aperture 700 and a second aperture 702, wherein the first aperture 700 is for receiving a guide probe 404 for pushing the expandable portion 109 out of the second aperture 702 of the housing 804, thereby guiding the expandable portion through the wound. As noted above, the force applied by the guide probe may also cause the seal to break (if present).

As noted above, in the double-walled example, a guide probe 404 may be used with the tamponade to guide the expandable portion into a wound. Thus, the handle mechanism for inserting the expandable portion into a wound may itself comprise a guide probe for guiding the expandable portion into the wound.

In some cases, the guide probe may be fixedly provided within the channel, such that the expandable portion is of a fixed length. In this case, the strut may open-up musculature of the wound to enable the expandable portion to be removed from the wound. This can be useful because otherwise the force of the musculature around the expandable tamponade may be such that it is difficult to remove the expandable portion from the wound without considerable force or causing damage.

Alternatively, as shown in Figures 7A to 7C, the guide probe 404 may be moveable within the channel, such that a length of the expandable portion 109 within a wound can be varied. This is particularly useful when the handle mechanism comprises a housing that contains the expandable portion. This is because the length of the guide probe used to push the expandable portion out of the housing can be varied, and thereby, the length of the expandable portion in a wound can be varied. This advantageously makes the tamponade adjustable to suit wounds of different sizes/depths. In this case, the strut may be inserted into the wound, before the expandable portion and guide probe is inserted into a wound, such that the strut opens-up musculature of the wound to enable the expandable portion to be inserted into and removed from the wound.

The flexible probe 404 may be coated with a double silicone layer. The dwelling locator tip 402 provides feedback to the user inserting the expandable tamponade 103 into a wound tract. This feedback allows the user to insert the expandable tamponade 103 as far into the wound tract as required, while the flexible probe helps with navigating complex wound topologies. The portion of the expandable portion 109 suitable for the length of the wound tract is released from the housing 804 during the insertion process. The housing may comprise an air release mechanism 143 and a connection to the hand-portable actuator 105, for example via tubing 414.

When the expandable tamponade 103 is inserted into a wound tract, the strut 806 of the expandable tamponade is first inserted before the flexible probe 404 pushes expandable portion 109 into the wound tract. The strut 806 of the expandable tamponade 103 is made from a stiff material that opens up a layer of musculature so that the inflatable portion 109 of the expandable tamponade, along with the flexible probe 404 and the dwelling locator tip 402, may be easily inserted into the wound tract. The strut 806 of the expandable tamponade 103 therefore functions to push back any layers of skin and/or musculature that might make it difficult for the expandable tamponade 103 to be inserted and helps keep the expandable tamponade 103 in place.

After the expandable tamponade 103 is inserted into the wound tract, the flexible probe 404 may be removed, as shown in Figure 7C and 7E, and the inflatable portion 109 of the expandable tamponade 103 may be inflated. The inflatable portion 109 may also be inflated with the probe 404 in place in the channel. Figures 7D and 7E show that the inflatable portion 109 of the expandable tamponade 103 comprises an inner balloon 808 and an outer balloon 810. That is, Figures 7D and 7E show the double-walled expandable portion. Both the inner and outer balloon can be, for example, made from medical grade silicone. The outer balloon 810 exerts pressure, for example, a pressure of 180mmHg, onto the tissue inside the wound. The inner balloon 808 provides a channel for receiving a guide probe 404. When the probe is removed, the inner balloon 808 may contract back into the housing as shown in Figure 7D.

An inspection probe may be inserted while the tamponade is inside a wound, to enable the wound to be inspected without destabilising the patient. Alternatively, the guide probe may itself comprise at least one sensing device. In either case, the sensing device of the inspection probe or guide probe may where the probe may be, for example, a sonography device, a camera, an infrared sensor and/or a temperature sensor. The sensing device may provide information about the wound that is relevant for treatment. Such information may include whether there is anything left in the wound (for example, shrapnel), whether there is any free fluid in the wound (for example, blood or intraperitoneal free fluid), and/or whether clotting has already taken place in the wound. Clotted blood has different properties than free blood, which is why it may be detectable with a sensor. The sensing device may enable a medical professional to locate the source of bleeding or see if any organs have been damaged. The sensing device may also be used to determine whether the patient should undergo further imaging, such as an **MRI** or CT scan.

The tamponade may be compatible with multiple different hand-held actuators and/or multiple different fluids for expanding the expandable portion. This is advantageous because a first responder may have a disposable and widely available actuator in their kit, whereas hospitals may have wider range of actuators and fluid sources. This means that a medical professional could keep the tamponade in a wound while attaching a different hand-held actuator or fluid source to increase the pressure applied by the tamponade.

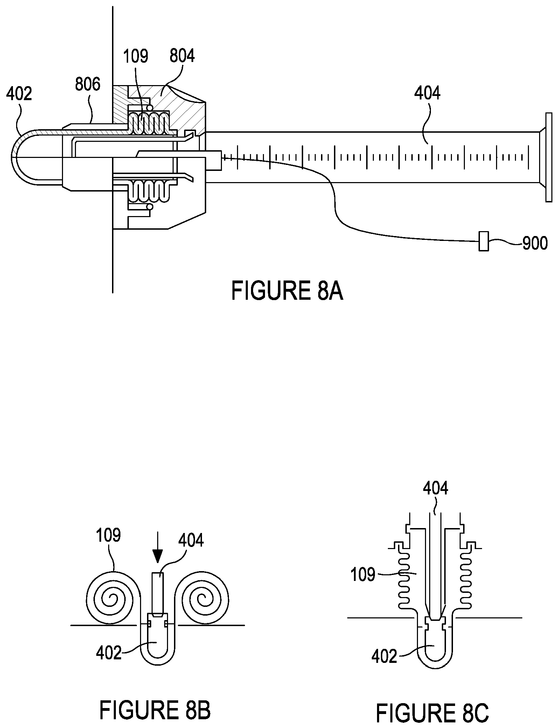

Figures 8A to 8C are sketches illustrating how the expandable portion 109 may be housed within the housing 804. Figure 8A shows a folded expandable portion 109 inside the housing 804, while Figure 8B shows the expandable portion in a rolled-up state inside the housing, and Figure 8C shows a similar arrangement to that of Figure 8A. The strut 806 of the expandable tamponade 103 also functions to prevent inflation of sections of the expandable portion 109 that have not been unrolled or unfolded. Thus, the expandable portion may be provided in the housing in a rolled, folded or wrapped state.

As shown in Figure 8C, the guide probe 404 may comprise markings 900 to indicate a depth of the wound into which the expandable portion is inserted. The markings 900 may be useful if, for example the length of a knife used to inflict the wound is known, so that the expandable tamponade 103 is not inserted any deeper than the length of the knife (as doing so could cause more damage). This may help medical personnel to understand the extent of the wound before the tamponade is removed, and whether the wound may have impacted any organs.

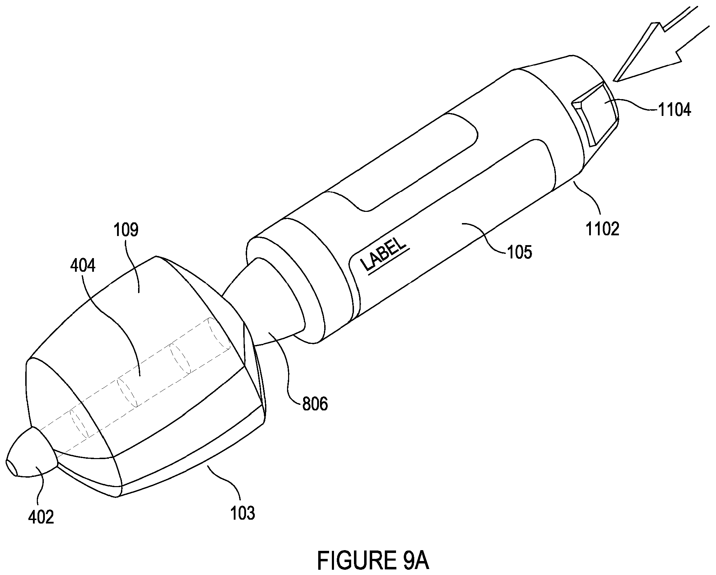

Figures 9A to 9C show an example arrangement of the expandable tamponade 103 and hand-portable actuator 105, which may be provided as a single device 1102 that can be separated into individual components during/after use. The actuator 105 may be a hand-held, mechanical actuator. Here, the strut 806 may be inserted into a wound. The actuator 105 comprises at least one button 1104, which may be coupled to a mechanism that causes a guide probe (and consequently, an expandable portion 109) to be pushed into a wound tract. This may also cause the expandable portion to be expanded, using fluid in the actuator 105. Alternatively, a further depress of button 1104 or a press of another button may cause the expandable portion to be expanded. The hand-held actuator 105 may be removed, while leaving the tamponade in position in the wound.

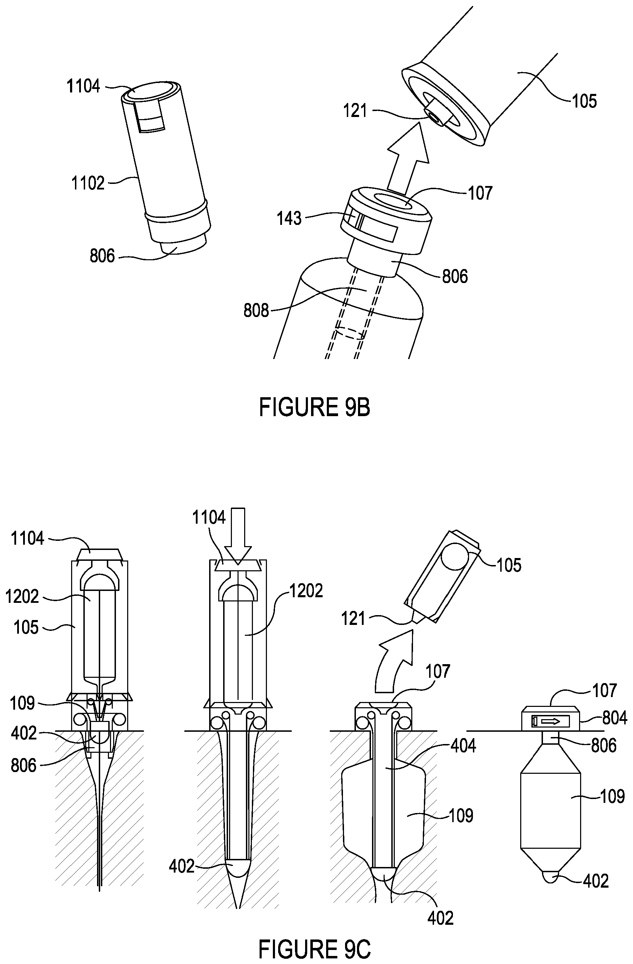

Figures 9B and 9C show how the device may be used, and additional features of the actuator and tamponade. Inflation of the inflatable portion 109 of the expandable tamponade 103 may happen through the use of, for example, a compressed gas contained in a canister 1202 within the actuator 105. The canister 1202 may contain any other suitable fluid for inflating the inflatable portion 109 of the expandable tamponade 103. The flexible probe 404 may be removed after inflation of the expandable tamponade 103, such that the channel can be used to insert an inspection probe, or the flexible probe 404 may remain inside the expandable tamponade 103. In some cases, the actuator 105 may simply be formed of a compressed gas canister that directly couples to the tamponade without any addition casing or mechanical components, buttons, and so on.

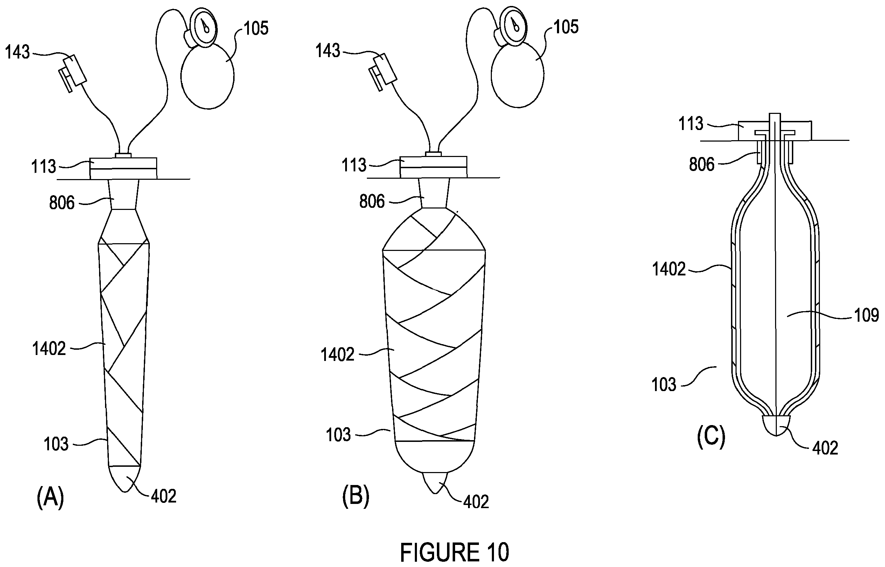

The canister 1202 may comprise a coupling means 121, while the housing 804 of the expandable tamponade 103 also comprises a coupling means 107 which may be disconnected from the coupling means 121 of the hand-portable actuator 105, as shown in Figure 10C.

Figures 10A to 10C show how the tamponade may comprise at least one layer of gauze around the expandable portion. Here, the inflatable portion 109 of the expandable tamponade 103 is wrapped in at least one layer of gauze in a way that protects the expandable portion but also enables the expandable portion to expand. The at least one layer of gauze may be formed of an absorbent material. The at least one layer of gauze may be impregnated with any one or more of: a haemostatic agent, an antihemorrhagic agent, and an infection mitigation compound (e.g. silver sulfadiazine). In this way, the surface of the expandable portion may encourage clotting and/or reduce infection. In the examples shown in Figures 10A to 10C, a guide probe may not be used to insert the expandable portion into a wound, because the at least one layer of gauze may provide sufficient stiffness for the expandable tamponade 103 to be inserted into the wound tract.

Additionally or alternatively, the tamponade may comprise a coating on an external surface of at least the expandable portion, wherein the coating comprises any one or more of: a haemostatic agent, an antihemorrhagic agent, and an infection mitigation compound (e.g. silver sulfadiazine). In this way, the surface of the expandable portion may encourage clotting and/or reduce infection. The coating may take the form of granules or particles embedded into or impregnated on the external surface, or may be a gel provided on the external surface.

In any case, the haemostatic component or agent may help to promote clotting inside the wound tract. This can be achieved, for example, by absorbing water content of the blood, which leads to clotting, or by absorbing large amounts of blood, which essentially forms a clot, or by otherwise creating a clotting reaction using a haemostatic agent. The haemostatic component or agent is brought into contact with free blood in the wound tract when the expandable tamponade 103 is inserted. When the inflatable portion 109 of the expandable tamponade 109 is inflated, it may exert pressure onto the wound tract and ensure that the haemostatic material or agent is in contact with the wound.

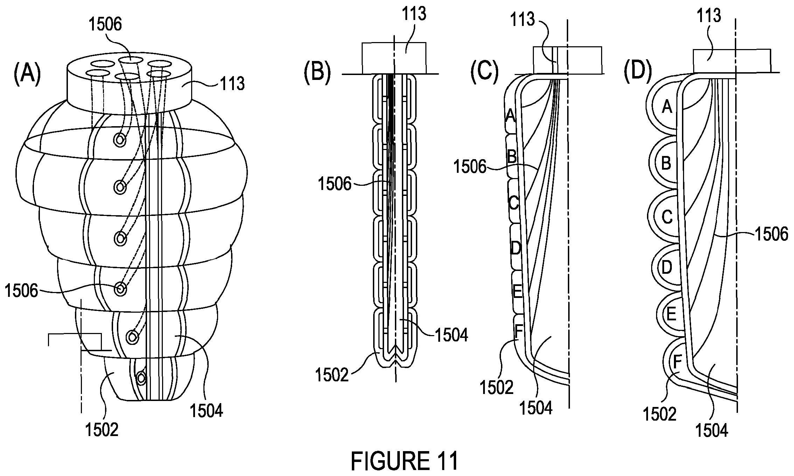

Figure 11 shows an expandable tamponade 103 comprising multiple inflatable chambers 1502 (labelled A to F). The tamponade may comprise a central inflatable portion 1504. Each inflatable chamber 1502 is inflated through a channel 1506. The inflatable chambers 1502 may all be inflated or only specific chambers may be inflated, for example, to exert pressure on a part of the wound tract that is haemorrhaging. That is, the expandable portion may comprise a plurality of sections, where each section is independently inflatable. This may advantageously enable the expandable portion to be inflated in a way that suits the shape of the wound.

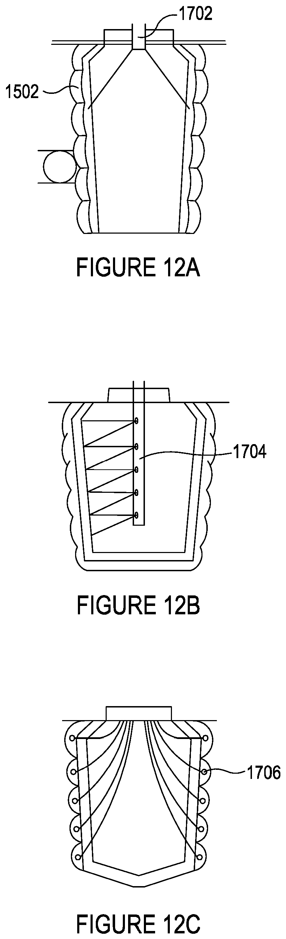

Figures 12A to 12C show how the tamponade may comprise at least one sensing device. Figure 12A shows a camera or imaging device 1702 may inserted into the expandable portion. The camera may be used to locate parts of the wound tract that are bleeding or otherwise inspect the wound. Figure 12B shows a temperature sensor 1704 being inserted into the expandable portion. The temperature sensor 1704 may be used to obtain more information about the wound tract and locate bleeding inside the wound tract. Clotted and free blood have different properties, and one of those different properties is their temperature. This is why a temperature sensor can be a simple and cost-effective way to obtain more information about a wound that the expandable tamponade 103 has been inserted into. Figure 12C shows the expandable portion 109 having at least one sensor 1706. The at least one sensor may be located on the outside of the inflatable portion 109 or the inflatable chambers 1502. The at least one sensor may also be located in any other part of the expandable tamponade 103 or expandable portion 109.

The information from any of the sensors may be used to help a medical professional determine which inflatable chamber or chambers 1502 should be inflated or inflated further to control the bleeding inside the wound tract. The decision about which of the inflatable chambers 1502 to inflate may also be made automatically, for example, by a processor of the hand-portable actuator 105. The hand-portable actuator 105 may then inflate the appropriate inflatable chambers 1502 automatically or it may display the information obtained from any of the sensors to a user on a user interface (where the interface may be either on the actuator 105 or on a device coupled to the actuator, such as a smartphone). The user may then make a decision on which of the inflatable chambers 1502 to inflate based on the information presented by the hand-portable actuator 105, or the user may decide to inflate different inflatable chambers 1502.

A decision on which inflatable chambers 1502 of the expandable tamponade 103 should be inflated may also be made based on external data. This external data may, for example, be obtained from a ballistic vest that is embedded with sensors. The ballistic vest may comprise an automatic injury detection sensor that may detect, report and profile, for example, projectile impacts. The expandable tamponade 103 or the hand-portable actuator 105 may be able to download and/or use this information to tailor the use of the expandable tamponade and/or the inflatable chambers 1502, for example to automatically inflate to a shape profile best suited to the wound site.

The expandable tamponade 103 may comprise a variety of different sensors at different locations within or on the outside of the expandable tamponade 103. The variety of sensor may provide first responders or healthcare professionals with information about the inside of the wound. This information may, for example, relate to whether or not blood is clotting or to the damage profile of the wound. The information may also relate to whether or not any objects are still left inside the wound, for example, shrapnel.

To help a first-aider or healthcare professional determine to what pressure to inflate the expandable tamponade 103 or the inflatable chambers 1502 should be inflated, there may also be provided a, for example, colour-coded chart showing different parts of the body. The first-aider or healthcare professional would then inflate the expandable tamponade 103 to the appropriate pressure for the body part in which the expandable tamponade 103 is placed.

Those skilled in the art will appreciate that while the foregoing has described what is considered to be the best mode and where appropriate other modes of performing present techniques, the present techniques should not be limited to the specific configurations and methods disclosed in this description of the preferred embodiment. Those skilled in the art will recognise that present techniques have a broad range of applications, and that the embodiments may take a wide range of modifications without departing from any inventive concept as defined in the appended claims.

## Claims

1. An expandable tamponade (103) for treating penetrating wounds, comprising:
an expandable portion (109) comprising:
a flexible outer wall (416a);
an inner wall (416b);
an inner chamber (418) between the flexible outer wall (416a) and the inner wall (416b), wherein the inner chamber (418) is fillable with fluid to expand the expandable portion (109) inside the wound (205); and
a channel defined by the inner wall (416b), for receiving a guide probe (404) or inspection probe;
a handle mechanism (406) for inserting the expandable portion into a wound (205), the handle mechanism (406) comprising:
a housing (804) containing the expandable portion (109) before the expandable portion (109) is inserted into the wound (205);
the guide probe (404) for guiding the expandable portion (109) into the wound (205), wherein the guide probe (404) is moveable within the channel, such that a length of the expandable portion (109) within a wound (205) can be varied;
coupling means (408) for coupling the expandable portion (109) to a fluid supply to expand the expandable portion (109) inside the wound (205), **characterised in that** the handle mechanism further comprises:
a strut (412) for insertion into the wound (205) before the insertion of the expandable portion and the guide probe (404) to open-up musculature of the wound (205), wherein the housing (804) and strut (412) surround at least part of the expandable portion when the expandable portion is inserted into the wound.

2. The tamponade as claimed in claim 1, wherein the expandable portion (109) is:
fixedly connected, at a first end, to the handle mechanism (406); and
fixedly connected, at a second end, to a rigid locator tip (402) for guiding the expandable portion (109) through the wound (205).

3. The tamponade as claimed in claim 1 wherein the inner wall (416b) is fixedly connected at a first end to the handle mechanism (406), and at a second end to a rigid locator tip (402) for guiding the expandable portion (109) through the wound (205).

4. The tamponade as claimed in claim 1 wherein the inner wall (416b) is fixedly connected at a first end to the handle mechanism (406), and wherein a second end of the inner wall is free to move within the inner chamber (418).

5. The tamponade as claimed in any preceding claim wherein the expandable portion (109) is configured to be contained within the housing (804) and the strut (412) of the handle mechanism (406).

6. The tamponade as claimed in any of claims 1 to 5 wherein the expandable portion (109) comprises a or the rigid locator tip (402) for guiding the expandable portion (109) through the wound (205), and wherein the rigid locator tip (402) protrudes through the strut (412) while the expandable portion (109) is configured to be contained within the housing (804).

7. The tamponade as claimed in any of claims 1 to 5 wherein the expandable portion (109) comprises a or the rigid locator tip (402) for guiding the expandable portion (109) through the wound (205), wherein the rigid locator tip (402) is configured to be contained within the housing (804) and/or the strut (412), and wherein the handle mechanism (406) comprises a seal that is configured to seal the strut (412).

8. The tamponade as claimed in any preceding claim wherein the expandable portion (109) is provided in the housing (804) in a rolled, folded or wrapped state; and/or wherein the housing (804) comprises a first aperture and a second aperture, wherein the first aperture is for receiving a guide probe (404) for pushing the expandable portion (109) out of the second aperture of the housing (804), thereby guiding the expandable portion (109) through the wound (205).

9. The tamponade as claimed in any preceding claim wherein the strut (412) is inserted into the wound (205), before the expandable portion (109) and guide probe (404) is inserted into a wound (205), wherein the strut (412) is configured to open-up musculature of the wound (205) to enable the expandable portion (109) to be inserted into and removed from the wound (205); and/or
further preferably, wherein the guide probe (404) comprises markings (900) to indicate a depth of the wound into which the expandable portion (109) is inserted; and/or
further preferably, wherein the guide probe (404) is removable after the expandable portion (109) has been inserted into a wound (205) and expanded.

10. The tamponade as claimed in any preceding claim wherein the guide probe (404) is flexible or rigid, and/or
wherein the guide probe (404) comprises at least one sensing device, preferably wherein the at least one sensing device is a sonography or ultrasound device.

11. The expandable tamponade (103) as claimed in any preceding claim wherein the at least one expandable tamponade (103) comprises an absorptive pad configured to absorb blood external to a wound (205), and/or wherein the at least one expandable tamponade (103) comprises sealing means configured to, at least partially, seal a wound site.

12. The tamponade as claimed in any preceding claim wherein the expandable portion comprises a plurality of sections, where each section is independently inflatable.

13. The tamponade as claimed in any preceding claim wherein the at least one expandable tamponade comprises an air release mechanism configured to enable air to be released into the atmosphere.

14. The tamponade as claimed in any preceding claim further comprising a coating on an external surface of the expandable portion, wherein the coating comprises any one or more of: a haemostatic agent, an antihemorrhagic agent, and an infection mitigation compound; and/or
wherein the tamponade comprises at least one layer of gauze around the expandable portion;
preferably, wherein the at least one layer of gauze is formed of an absorbent material.

## Patentansprüche

1. Expandierbare Tamponade (103) zur Behandlung penetrierender Wunden, umfassend:
einen expandierbaren Anteil (109), umfassend:
eine flexible Außenwand (416a);
eine Innenwand (416b);
eine Innenkammer (418) zwischen der flexiblen Außenwand (416a) und der Innenwand (416b), wobei die Innenkammer (418) mit Fluid füllbar ist, um den expandierbaren Anteil (109) innerhalb der Wunde (205) zu expandieren; und
einen durch die Innenwand (416b) definierten Kanal zur Aufnahme einer Führungssonde (404) oder Inspektionssonde;
einen Griffmechanismus (406) zum Einführen des expandierbaren Anteils in eine Wunde (205), wobei der Griffmechanismus (406) umfasst:
ein Gehäuse (804), das den expandierbaren Anteil (109) enthält, bevor der expandierbare Anteil (109) in die Wunde (205) eingeführt wird;
die Führungssonde (404) zum Führen des expandierbaren Anteils (109) in die Wunde (205), wobei die Führungssonde (404) innerhalb des Kanals beweglich ist, so dass eine Länge des expandierbaren Anteils (109) innerhalb einer Wunde (205) variiert werden kann;
Kopplungsmittel (408) zum Koppeln des expandierbaren Anteils (109) an eine Fluidzufuhr zum Expandieren des expandierbaren Anteils (109) innerhalb der Wunde (205), **dadurch gekennzeichnet, dass** der Griffmechanismus weiter umfasst:
eine Spreize (412) zum Einführen in die Wunde (205) vor dem Einführen des expandierbaren Anteils und der Führungssonde (404), um die Muskulatur der Wunde (205) zu öffnen, wobei das Gehäuse (804) und die Spreize (412) mindestens einen Teil des expandierbaren Anteils umgeben, wenn der expandierbare Anteil in die Wunde eingeführt wird.

2. Tamponade nach Anspruch 1, wobei der expandierbare Anteil (109) Folgendes ist:
an einem ersten Ende fest mit dem Griffmechanismus (406) verbunden; und
an einem zweiten Ende fest mit einer starren Ortungsspitze (402) verbunden, um den expandierbaren Anteil (109) durch die Wunde (205) zu führen.

3. Tamponade nach Anspruch 1, wobei die Innenwand (416b) an einem ersten Ende fest mit dem Griffmechanismus (406) und an einem zweiten Ende mit einer starren Ortungsspitze (402) verbunden ist, um den expandierbaren Anteil (109) durch die Wunde (205) zu führen.

4. Tamponade nach Anspruch 1, wobei die Innenwand (416b) an einem ersten Ende fest mit dem Griffmechanismus (406) verbunden ist, und wobei ein zweites Ende der Innenwand sich innerhalb der Innenkammer (418) frei bewegen kann.

5. Tamponade nach einem der vorangehenden Ansprüche, wobei der expandierbare Anteil (109) so ausgestaltet ist, dass er in dem Gehäuse (804) und der Spreize (412) des Griffmechanismus (406) enthalten ist.

6. Tamponade nach einem der Ansprüche 1 bis 5, wobei der expandierbare Anteil (109) eine oder die starre Ortungsspitze (402) zum Führen des expandierbaren Anteils (109) durch die Wunde (205) umfasst, und wobei die starre Ortungsspitze (402) durch die Spreize (412) hindurch vorspringt, während der expandierbare Anteil (109) so ausgestaltet ist, dass er im Gehäuse (804) enthalten ist.

7. Tamponade nach einem der Ansprüche 1 bis 5, wobei der expandierbare Anteil (109) eine oder die starre Ortungsspitze (402) zum Führen des expandierbaren Anteils (109) durch die Wunde (205) umfasst, wobei die starre Ortungsspitze (402) so ausgestaltet ist, dass sie in dem Gehäuse (804) und/oder der Spreize (412) enthalten ist, und wobei der Griffmechanismus (406) eine Dichtung umfasst, die zur Abdichtung der Spreize (412) ausgestaltet ist.

8. Tamponade nach einem der vorangehenden Ansprüche, wobei der expandierbare Anteil (109) in dem Gehäuse (804) in einem gerollten, gefalteten oder gewickelten Zustand bereitgestellt wird; und/oder wobei das Gehäuse (804) eine erste Öffnung und eine zweite Öffnung umfasst, wobei die erste Öffnung zum Aufnehmen einer Führungssonde (404) zum Herausschieben des expandierbaren Anteils (109) aus der zweiten Öffnung des Gehäuses (804) ist, wodurch der expandierbare Anteil (109) durch die Wunde (205) geführt wird.

9. Tamponade nach einem der vorangehenden Ansprüche, wobei die Spreize (412) in die Wunde (205) eingeführt wird, bevor der expandierbare Anteil (109) und die Führungssonde (404) in eine Wunde (205) eingeführt werden, wobei die Spreize (412) so ausgestaltet ist, dass sie die Muskelstruktur der Wunde (205) öffnet, damit der expandierbare Anteil (109) in die Wunde (205) eingeführt und daraus entfernt werden kann; und/oder
wobei weiter bevorzugt die Führungssonde (404) Markierungen (900) umfasst, um eine Tiefe der Wunde anzugeben, in die der expandierbare Anteil (109) eingeführt worden ist; und/oder
wobei weiter bevorzugt die Führungssonde (404) entfernbar ist, nachdem der expandierbare Anteil (109) in eine Wunde (205) eingeführt und expandiert worden ist.

10. Tamponade nach einem der vorangehenden Ansprüche, wobei die Führungssonde (404) flexibel oder starr ist und/oder
wobei die Führungssonde (404) mindestens eine Abfühlvorrichtung umfasst, wobei vorzugsweise die mindestens eine Abfühlvorrichtung eine Sonographie- oder Ultraschallvorrichtung ist.

11. Expandierbare Tamponade (103) nach einem der vorangehenden Ansprüche, wobei die mindestens eine expandierbare Tamponade (103) ein Absorptionspad umfasst, das ausgestaltet ist, um Blut außerhalb einer Wunde (205) zu absorbieren, und/oder wobei die mindestens eine expandierbare Tamponade (103) Dichtungsmittel umfasst, die so ausgestaltet sind, dass sie eine Wundstelle mindestens teilweise abdichten.

12. Tamponade nach einem der vorangehenden Ansprüche, wobei der expandierbare Anteil eine Vielzahl von Segmenten umfasst, wobei jedes Segment unabhängig befüllbar ist.

13. Tamponade nach einem der vorangehenden Ansprüche, wobei die mindestens eine expandierbare Tamponade einen Luftablassmechanismus umfasst, der so ausgestaltet ist, dass Luft in die Atmosphäre abgelassen werden kann.

14. Tamponade nach einem der vorangehenden Ansprüche, die ferner eine Beschichtung auf einer Außenfläche des expandierbaren Anteils umfasst, wobei die Beschichtung eines oder mehrere von einem Hämostatikum, einem Antihämorrhagikum und einer infektionsmindernden Verbindung umfasst; und/oder
wobei die Tamponade mindestens eine Schicht aus Gaze um den expandierbaren Anteil herum umfasst;
wobei vorzugsweise die mindestens eine Schicht Gaze aus einem Absorbensmaterial gebildet ist.

## Revendications

1. Tamponnement expansible (103) pour le traitement de plaies pénétrantes, comprenant :
une partie expansible (109) comprenant :
une paroi extérieure flexible (416a) ;
une paroi intérieure (416b) ;
une chambre intérieure (418) entre la paroi extérieure flexible (416a) et la paroi intérieure (416b), la chambre intérieure (418) pouvant être remplie d'un fluide pour dilater la partie expansible (109) à l'intérieur de la plaie (205) ; et
un canal défini par la paroi intérieure (416b), destiné à recevoir une sonde de guidage (404) ou une sonde d'inspection ;
un mécanisme de poignée (406) pour insérer la partie expansible dans une plaie (205), le mécanisme de poignée (406) comprenant :
un logement (804) contenant la partie expansible (109) avant que la partie expansible (109) ne soit insérée dans la plaie (205) ;
la sonde de guidage (404) pour guider la partie expansible (109) dans la plaie (205), la sonde de guidage (404) étant mobile dans le canal, de sorte qu'une longueur de la partie expansible (109) dans une plaie (205) peut être modifiée ;
un moyen de couplage (408) pour coupler la partie expansible (109) à une alimentation en fluide pour dilater la partie expansible (109) à l'intérieur de la plaie (205), **caractérisé en ce que** le mécanisme de poignée comprend en outre :
une entretoise (412) destinée à être insérée dans la plaie (205) avant l'insertion de la partie expansible et de la sonde de guidage (404) pour ouvrir la musculature de la plaie (205), le logement (804) et l'entretoise (412) entourant au moins une partie de la partie expansible lorsque la partie expansible est insérée dans la plaie.

2. Tamponnement selon la revendication 1, la partie expansible (109) étant :
reliée de manière fixe, au niveau d'une première extrémité, au mécanisme de poignée (406) ; et
reliée de manière fixe, au niveau d'une seconde extrémité, à une pointe de positionnement rigide (402) pour guider la partie expansible (109) à travers la plaie (205).

3. Tamponnement selon la revendication 1, la paroi intérieure (416b) étant reliée de manière fixe au niveau d'une première extrémité au mécanisme de poignée (406), et au niveau d'une seconde extrémité à une pointe de positionnement rigide (402) pour guider la partie expansible (109) à travers la plaie (205).

4. Tamponnement selon la revendication 1, la paroi intérieure (416b) étant reliée de manière fixe au niveau d'une première extrémité au mécanisme de poignée (406), et une seconde extrémité de la paroi intérieure étant libre de se déplacer dans la chambre intérieure (418).

5. Tamponnement selon l'une quelconque des revendications précédentes, la partie expansible (109) étant configurée pour être contenue dans le logement (804) et l'entretoise (412) du mécanisme de poignée (406).

6. Tamponnement selon l'une quelconque des revendications 1 à 5, la partie expansible (109) comprenant une ou la pointe de positionnement rigide (402) pour guider la partie expansible (109) à travers la plaie (205), et la pointe de positionnement rigide (402) faisant saillie à travers l'entretoise (412) tandis que la partie expansible (109) est configurée pour être contenue dans le logement (804).

7. Tamponnement selon l'une quelconque des revendications 1 à 5, la partie expansible (109) comprenant une ou la pointe de positionnement rigide (402) pour guider la partie expansible (109) à travers la plaie (205), la pointe de positionnement rigide (402) étant configurée pour être contenue dans le logement (804) et/ou l'entretoise (412), et le mécanisme de poignée (406) comprenant un joint qui est configuré pour sceller l'entretoise (412).

8. Tamponnement selon l'une quelconque des revendications précédentes, la partie expansible (109) étant prévue dans le logement (804) dans un état roulé, plié ou enroulé ; et/ou le logement (804) comprenant une première ouverture et une seconde ouverture, la première ouverture étant destinée à recevoir une sonde de guidage (404) pour pousser la partie expansible (109) hors de la seconde ouverture du logement (804), guidant ainsi la partie expansible (109) à travers la plaie (205).

9. Tamponnement selon l'une quelconque des revendications précédentes, l'entretoise (412) étant insérée dans la plaie (205), avant que la partie expansible (109) et la sonde de guidage (404) soient insérées dans une plaie (205), l'entretoise (412) étant configurée pour ouvrir la musculature de la plaie (205) pour permettre à la partie expansible (109) d'être insérée dans la plaie (205) et retirée de celle-ci ; et/ou
de préférence encore, la sonde de guidage (404) comprenant des repères (900) indiquant une profondeur de la plaie dans laquelle la partie expansible (109) est insérée ; et/ou
de préférence encore, la sonde de guidage (404) pouvant être retirée après que la partie expansible (109) a été insérée dans une plaie (205) et dilatée.

10. Tamponnement selon l'une quelconque des revendications précédentes, la sonde de guidage (404) étant flexible ou rigide, et/ou
la sonde de guidage (404) comprenant au moins un dispositif de détection, de préférence l'au moins un dispositif de détection étant un dispositif d'échographie ou à ultrasons.

11. Tamponnement expansible (103) selon l'une quelconque des revendications précédentes, l'au moins un tamponnement expansible (103) comprenant un tampon absorbant configuré pour absorber du sang externe à une plaie (205), et/ou l'au moins un tamponnement expansible (103) comprenant un moyen d'étanchéité configuré pour, au moins partiellement, sceller un site de plaie.

12. Tamponnement selon l'une quelconque des revendications précédentes, la partie expansible comprenant une pluralité de sections, chaque section étant gonflable indépendamment.

13. Tamponnement selon l'une quelconque des revendications précédentes, l'au moins un tamponnement expansible comprenant un mécanisme de libération d'air configuré pour permettre à l'air d'être libéré dans l'atmosphère.

14. Tamponnement selon l'une quelconque des revendications précédentes, comprenant en outre un revêtement sur une surface externe de la partie expansible, le revêtement comprenant l'un quelconque ou plusieurs parmi : un agent hémostatique, un agent antihémorragique, et un composé atténuant l'infection ; et/ou
le tamponnement comprenant au moins une couche de gaze autour de la partie expansible ;
de préférence, l'au moins une couche de gaze étant formées d'un matériau absorbant.
